# EUROPEAN PATENT SPECIFICATION

(11) **EP 3 807 243 B1**
(45) Date of publication and mention of the grant of the patent: **22.05.2024**
(21) Application number: 19732261.3
(22) Date of filing: 10.06.2019
(51) Int. Cl.: C07C 213/00, C07C 213/08

(54) **A NOVEL PROCESS FOR THE PREPARATION OF TAPENTADOL**
NEUARTIGES VERFAHREN ZUR HERSTELLUNG VON TAPENTADOL
NOUVEAU PROCÉDÉ DE PRÉPARATION DE TAPENTADOL

(30) Priority: 15.06.2018 IN 201831022432
(43) Date of publication of application: 21.04.2021
(73) Proprietor: Pharmathen S.A., 15351 Pallini Attikis (GR)
(72) Inventor: KOFTIS, V. Theocharis, 57001 Thessaloniki (GR); NEOKOSMIDIS, Efstratios, 57001 Thessaloniki (GR); STATHAKIS, Christos, 57001 Thessaloniki (GR); GKIZIS, Petros, 57001 Thessaloniki (GR); PANAGIOTIDIS, Theodoros, 57001 Thessaloniki (GR)
(86) International application number: PCT/EP2019/025173
(87) International publication number: WO 2019/238267

(56) References cited:
- WO-A1-2005/000788
- WO-A1-2008/012283
- US-A1- 2016 060 197

## Description

### TECHNICAL FIELD OF THE INVENTION

The present invention relates to a novel process for the preparation of Tapentadol and intermediate thereof.

### BACKGROUND OF THE INVENTION

Tapentadol is the INN (International Non-proprietary Name) of 3-[(IR, 2R)-3-(dimethylamino)-1-ethyl-2-methylpropyl]phenol monohydrochloride, represented by the formula:

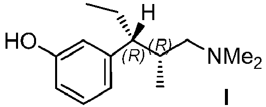

The chemical structure of tapentadol has been disclosed in EP-A-0693475 as compound (+21). The synthesis of tapentadol is described in Example 1 and Example 24 steps 1 to 3.

There are many synthetic procedures, besides the above, disclosed in the prior art for the preparation of Tapentadol or its key intermediate, compound of formula IIa.

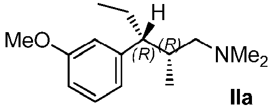

PCT publication WO2008012283A1 discloses a process for the preparation of (2R, 3R)-3-(-methoxyphenyl)-N,N,2-trimethylpentanamine (compound of formula II), by treating corresponding hydroxyl compound of formula **IIIa** with trifluoroacetic acid anhydride or acetyl chloride or ethyl oxalyl chloride and subsequent hydrogenation with a transition metal catalyst such as Palladium on carbon, to produce (2*R*,3*R*)-3-(3-methoxyphenyl)-*N,N*,2-trimethylpentanamine (denoted as compound of formula **IIa**) or its acid addition salts (compound of formula **IIa'**), as shown in scheme 1. Compound of formula **IIa'** is then converted to Tapentadol by demethylation according to methods disclosed in the prior art (see for example EP-A-0693475). 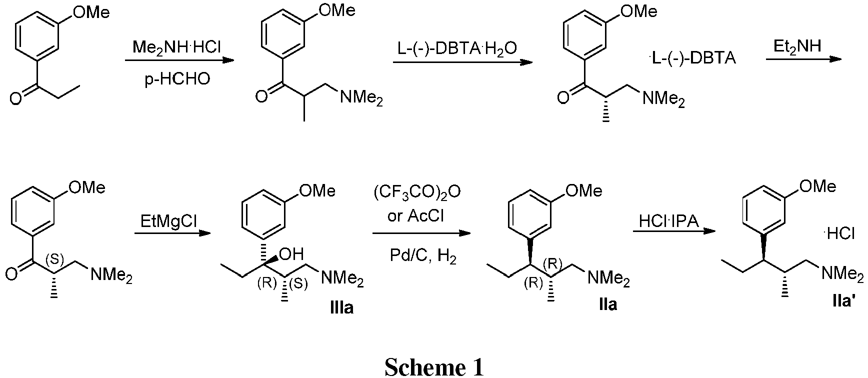 Indian patent application IN201641017954 discloses an alternative synthesis, which utilizes compound of formula **IIIb** to prepare compound of formula **IIa.** Compound of formula **IIIb** is prepared according to methods of prior art, in particular the ones disclosed in USRE39593, US6344558B2 and WO2012101649.

According to the patent application, (2S,3S)-1-dimethylamino-3-(3-methoxyphenyl)-2-methyl-pentan-3-ol (the *S,S*-isomer of compound of formula **III**, denoted as **IIIb**) with a hydrosilane reagent in presence of a suitable acid to produce a diastereomeric mixture of 2*R*,3*R* and 2R,3S-[3-(3-methoxyphenyl)-2-methylpentyl]dimethylamine hydrochloride (denoted as compounds of formulae **IIa** and **IIb**) according to scheme 2. The diastereomeric mixture is converted to the respective hydrochloride salts which allows for the separation of the desired 2*R*,3*R* diastereomer IIa. The separation is disclosed to be preferably performed by means of fractional crystallization, which may be performed more than once in order to achieve the desired diastereomeric purity. Demethylation then affords Tapentadol either as a free base or as a pharmaceutically acceptable salt. Notably, this strategy employs compound of formula IIIb as a starting material, which is the *S,S*-diastereomer of compound of formula III. This diastereomer, although accessible according to the prior art procedures mentioned above, is prepared by procedures which are more complicated and with considerably lower yield compared to the ones that allow access to diastereomer IIIa.

It would thus be desirable to prepare Tapentadol without the need to use expensive reagents such as the ones required for hydrogenation or without using expensive starting materials.

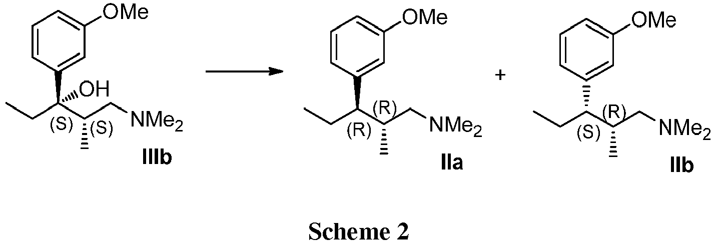

### DEFINITIONS

The following terms shall have, for the purposes of this application, including the claims appended hereto, the respective meanings set forth below. It should be understood that when reference herein is made to a general term, such as acid, base, salt, etc. one skilled in the field may make appropriate selections for such reagents from those given in the definitions below, as well as from additional reagents recited in the specification that follows, or from those found in literature references in the field.

"Acid" refers to any compound that contains hydrogen and dissociates in water or solvent to produce positive hydrogen ions, as well as Lewis acids, including but not limited to acids such as hydrochloric acid, hydrobromic acid, sulfuric acid, nitric acid, phosphoric acid, acetic acid, trihaloacetic acid (e.g., trifluoroacetic acid), maleic acid, sulfonic acids such as methanesulfonic acids, toluenesulfonic acids and camphorsulfonic acids, propionic acids such as (R)-chloropropionic acid, phthalamic acids such as N-[(R)-1-(1-naphthyl) ethyl]phthalamic acid, mandelic acid, tartaric acids such as D- or L-tartaric acid, and its derivatives, such as diaroyl tartaric acids, lactic acids, camphoric acids, aspartic acids, citronellic acids, and so forth. Thus, the term includes weak acids such as ethanoic acid and hydrogen sulfide; strong organic acids such as methanesulfonic acid, trifluoroacetic acid, and so forth.

"Protic acid" refers to an acid which, when dissolved in water, liberates H⁺ ions.

"Chiral acid" refers to an acid which is also a chiral compound, i.e. a compound that contains an asymmetric center (chiral atom or chiral center) and thus can occur in two nonsuperimposable mirror-image forms (enantiomers). Common examples of chiral acids are (1*R*)- and (1*S*)- camphorsulfonic acid, (*R*)- and (*S*)-chloropropionic acid, N-[(*R*)- and (*S*)-1-(1-naphthyl) ethyl]phthalamic acid, *R*)- and (*S*)-mandelic acid, D- and L-tartaric acid and its derivatives, such as diaroyl tartaric acids, D- and L-lactic acid, all diastereomers of camphoric acids, D- and L-aspartic acids and so forth.

"Achiral acid" accordingly refers to acids as defined generally above, that are not chiral compounds, as for example hydrochloric acid, hydrobromic acid, sulfuric acid, nitric acid, phosphoric acid, acetic acid, trihaloacetic acid (e.g., trifluoroacetic acid), maleic acid, sulfonic acids such as methanesulfonic acids, toluenesulfonic acids and so forth.

"Lewis acid" is defined herein as any chemical species that is an electron-pair acceptor, i.e., any chemical species that is capable of receiving an electron pair, without limitation. The Lewis acid (also referred to as the Lewis acid catalyst) may be any Lewis acid based on transition metals, lathanoid metals, and metals from Group 4, 5, 13, 14 and 15 of the periodic table of the elements, including boron, aluminum, gallium, indium, titanium, zirconium, tin, vanadium, arsenic, antimony, bismuth, lanthanum, dysprosium, and ytterbium. Non-limiting examples of Lewis acids are titanium tetrachloride, titanium tetrabromide, titanium isopropoxide, aluminium chloride, aluminium bromide, aluminium isopropoxide, boron trifluoride, boron tribromide, tin tetrachloride, tin tetrabromide, stannous chloride, zinc chloride, iron trichloride, iron tribromide and their complexes thereof.

Acceptable salts of the compounds prepared herein include suitable acid addition salts thereof. They are referred to as "acid addition salts" or simply "salts". Salts are formed, for example, with strong acids such as mineral acids, e. g. sulphuric acid, phosphoric acid, nitric acid, boric acid or hydrohalic acids; with strong organic carboxylic acids, such as alkanecarboxylic acids of 1 to 4 carbon atoms which are unsubstituted or substituted (e. g., by halogen), such as acetic acid and trifluoroacetic acid; with saturated or unsaturated dicarboxylic acids, for example oxalic, malonic, succinic, maleic, fumaric, phthalic or tetraphthalic; with hydroxycarboxylic acids, for example ascorbic, glycolic, lactic, malic, tartaric or citric acid and their esters, eg diaroyl tartaric acids; with aminoacids, for example aspartic or glutamic acid; with benzoic acid; or with organic sulfonic acids, such as (C1-4)-alkyl- or aryl-sulfonic acids which are substituted or unsubstituted (for example, by a halogen) such as methane- or p-toluene sulfonic acid.

Pharmaceutically acceptable salts are salts that retain the desired biological activity of the parent compound and do not impart undesired toxicological effects. Examples of such salts are (a) acid addition salts formed with inorganic acids, for example hydrochloric acid, hydrobromic acid, sulfuric acid, phosphoric acid, nitric acid and the like; and salts formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, palmitic acid, alginic acid, polyglutamic acid, naphthalenesulfonic acid, methanesulfonic acid, p-toluenesulfonic acid, naphthalenedisul fonic acid, polygalacturonic acid, and the like; (b) salts formed from elemental anions such as chlorine, bromine, and iodine, and (c) salts derived from bases, such as ammonium salts, alkali metal salts such as those of sodium and potassium, alkaline earth metal salts such as those of calcium and magnesium, and salts with organic bases such as dicyclohexylamine and N-methyl-D-glucamine. A review of suitable pharmaceutical salts may be found in Berge et al., J. Pharm. Sci., 66, 1, 19 (1977).

### DETAILED DESCRIPTION OF THE INVENTION

In a first embodiment, the present invention relates to a process for the preparation of a compound of formula IIa or salt thereof from compound of formula IIIa or a salt thereof, comprising the steps of:
a) performing deoxygenation of compound of formula IIIa or a salt thereof in the presence of a hydrosilane reagent and an acid to provide compound of formula IIa, optionally in the form of a diastereomeric mixture; 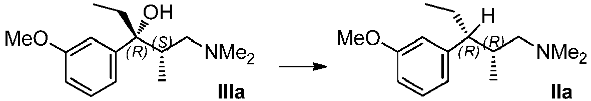
b) optionally treating the product of step a) with a chiral or achiral acid or a mixture thereof, forming an acid addition salt via the amine group, and separating the salt of the desired (2*R*, 3*R*)-[3-(3-methoxyphenyl)-2-methyl-pentyl]dimethylamine (compound of formula IIa).

Compound of formula IIIa or its salt is prepared according to prior art procedures, as disclosed for example in EP2046724 (or the respective WO2008012047). Compound of formula IIIa, ie the 2*S*, 3*R* diastereomer of compound of formula III, is accessible in a reliable and reproducible manner, as extensively shown in said prior art disclosures. It is also commercially available either as a free base or in the form of an acid addition salt.

The deoxygenation performed in step a typically employs a hydrosilane reducing agent, which may be selected from triethylsilane, trimethylsilane, dimethylphenylsilane, phenyl silane, triphenylsilane, trichlorosilane, tris(trimethylsilyl)silane, polymethylhydrosiloxane.

In order for the deoxygenation to be performed, the hydrosilane reducing agent requires also the presence of an acid (hereinafter referred to as acid of step a). The acid of step a may be any acid capable of activating compound of formula IIIa. The acid may be a Lewis acid or a protic acid. In the case of Lewis acids, the acid may be selected from titanium tetrachloride, aluminium chloride, aluminium bromide, boron trifluoride, boron tribromide, tin tetrachloride, tin tetrabromide, stannous chloride, ferric chloride, zinc chloride. In the case of a protic acid, the acid may be selected from trifluoroacetic acid, p-toluolosulfonic acid or methanesulfonic acid.

The solvent of the reaction may be selected from hydrocarbons, such as toluene, xylenes, halogenated hydrocarbons, such as dichloromethane, 1,2 dichloroethane, chloroform, chlorobenzene, dichlorobenzene. Preferable is dichloromethane.

The temperature may range from about (-50) °C to boiling point of the solvent of the reaction. Preferable temperature range is (-10)-50 °C. More preferable is 0-25 °C. Even more is preferable 5-10°C.

The hydrosilane used may range from 1.5 to 10 equivalents. Preferably, the equivalents used may be 2.0.

The acid used may range from 2 to 10 equivalents. Preferably, the equivalents used may be 2.1.

The deoxygenation reaction converts compound of formula IIIa into compound of formula IIa. Compound of formula IIa may be optionally formed as part of a diastereomeric mixture, ie a mixture of compound of formula IIa and its diastereomer, compound of formula IIb.

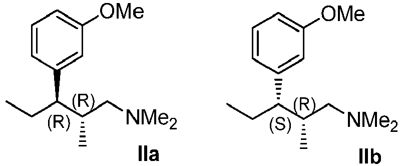

The ratio of the two diastereomers depends on various factors such as the temperature of the reaction and the solvent.

Where the diastereomeric purity is not satisfactory, the diastereomeric mixture may be subjected to treatment with an acid, upon which the acid addition salt formed allows separation of the two diastereomers.

The acid used in step b is selected from chiral or achiral acids and may be any of the following: hydrochloric acid, hydrobromic acid, nitric acid, oxalic acid, succinic acid, maleic acid, fumaric acid, sulfuric acid, phosphoric acid, acetic acid, priopionic acid, benzenesulfonic acid, toluenesulfonic acid, citric acid, tartaric acid and its derivatives, malic acid, mandelic acid and its derivatives, camphorosulfonic acid and its derivatives. Preferable acids are hydrochloric acid, hydrobromic acid, sulfuric acid, benzenesulfonic acid, toluenesulfonic acid, tartaric acid and its derivatives and mandelic acid.

The acid selected to effect the separation of diastereomers may vary and depend on the diastereomeric ratio of the diastereomeric mixture produced in step a. In addition, step b may be performed more than once and the acid used in each cycle may not necessarily be the same.

The solvent that may be used in step b depends on the selection of the acid used to effect the separation of the diastereomers. In general, commonly used polar organic solvents or water are suitable, such as ketones, alchohols, esters, ethers, halogenated hydrocarbons and mixtures thereof. In case of hydrohalic acid salts, ketone solvents are preferable, such as acetone, butanone, methyl isobutyl ketone. On the other hand, in case of tartaric acid derivatives' addition salts, aqueous alcoholic solvents are preferable.

The appropriate solvent of step b depends on the selection of the acid used to effect the separation of diastereomers. For instance, for the separation of a diastereomeric mixture of HCl addition salts of IIa the solvent is selected among acetone, butanone-2, methyl isobutyl ketone and related ketones. On the other hand for the separation of a mixture of tartaric acid derivatives addition salts the most appropriate solvent is aqueous methanol, aqueous ethanol or similar solvents.

The resolution of the diastereomeric mixture may generally be performed according to well-established prior art procedures, such as the one using hydrochloride salts in WO2008012047 or the one using diaroyl tartaric acid derivatives in WO2016023913 or according to the present invention.

In a preferred embodiment the deoxygenation reaction of compound of formula IIIa into compound of formula IIa, as describe in step a, results in a diastereomeric mixture which is then separated as described in step b to provide compound of formula IIa.

Compound of formula IIIa may be employed either as the free amine base, or as its acid addition salt. In a preferred embodiment, compound of formula IIIa is in the form of its acid addition salt. The counter ion of the acid addition salt may originate from an acid, as defined above. Preferably, the acid is selected from hydrochloric acid, hydrobromic acid, sulfuric acid, nitric acid, phosphoric acid, acetic acid, trihaloacetic acid (e.g., trifluoroacetic acid), maleic acid, sulfonic acids such as methanesulfonic acids, toluenesulfonic acids and camphorsulfonic acids, propionic acids such as (R)-chloropropionic acid, phthalamic acids such as N-[(R)-1-(1-naphthyl) ethyl]phthalamic acid, tartaric acids such as L-tartaric acid and dibenzyl-L-tartaric acid, lactic acids, camphoric acids, aspartic acids, citronellic acids. More preferable are hydrochloric and hydrobromic acid.

In a second embodiment of the present invention, there is provided a process for the preparation of Tapentadol or a pharmaceutically acceptable salt thereof, comprising steps a and b as defined in previous embodiments and further comprising demethylating compound of formula IIa, to provide compound of formula I and optional conversion of compound of formula I into a pharmaceutivally acceptable salt thereof.

The demethylation reaction may be performed according to methods disclosed in the prior art. The reagents used therein include but are not limited to hydrobromic acid, methanesulfonic acid, hydrochloric acid, trifluoroacetic acid, aluminium chloride, aluminium bromide, or combination thereof. Hydrobromic acid is preferable.

### EXAMPLES

### EXAMPLE 1:

50 mg of (2S, 3R)-1-(Dimethylamino)-3-(3-methoxyphenyl)-2-methylpentan-3-ol are charged in 10 ml RB flask, along with 1.0 ml dichloromethane. 0.32 ml triethylsilane are added under stirring and the mixture is cooled to 0-5 °C. 67 mg aluminium trichloride are added at this temperature and the mixture is stirred for 2 hours at 0-5 °C. Mixture is then allowed to reach ambient temperature and further stirred for 2 hours.

Mixture is cooled down again to 0-5 °C and diluted with 3.0 ml of ethyl acetate, quenched with 2.0 ml of 10% w/v aq. solution of sodium hydroxide and allowed to reach ambient temperature again. The organic phase is separated and the aqueous one is extracted with another portion of 3.0 ml of ethyl acetate. Organic phase is separated again and combined with the first one, to be dried over sodium sulfate. After filtration, solvents are distilled off to provide 55 mg of crude product.

### EXAMPLE 2:

50 mg of (2*S*, 3*R*)-1-(Dimethylamino)-3-(3-methoxyphenyl)-2-methylpentan-3-ol are charged in 10 ml RB flask, along with 1.0 ml toluene. 0.32 ml triethylsilane are added under stirring and the mixture is cooled to 0-5 °C. 67 mg aluminium trichloride are added at this temperature and the mixture is stirred for 2 hours at 0-5 °C. Mixture is then allowed to reach ambient temperature and further stirred for 2 hours.

Mixture is subjected to workup as above, to provide 57 mg of crude product.

### EXAMPLE 3:

250 mg of (2*S*, 3*R*)-1-(Dimethylamino)-3-(3-methoxyphenyl)-2-methylpentan-3-ol are charged in a 10ml RB flask, along with 10 ml dichloromethane. The mixture is then cooled under stirring to 0-5 °C. At this temperature 0.5 ml triethylsilane are added, followed by 0.76 ml trifluoroacetic acid. Mixture is warmed to room temperature and stirring is continued for 1 hour. Mixture is heated at about 50 °C and temperature is maintained for 2 hours.

Mixture is subjected to workup as above, to provide 223 mg of crude product.

### EXAMPLE 4:

250 mg of (2*S*, 3*R*)-1-(Dimethylamino)-3-(3-methoxyphenyl)-2-methylpentan-3-ol are charged in a 10ml RB flask, along with 10 ml dichloromethane. The mixture is then cooled under stirring to 0-5 °C. At this temperature 0.5 ml triethylsilane are added, followed by 1.23 ml boron trifluoride etherate. Mixture is warmed to room temperature and stirring is continued for 1 hour. Mixture is heated at about 50 °C and temperature is maintained for 2 hours. Mixture is subjected to workup as above, to provide 238 mg of crude product.

### EXAMPLE 5:

A 1L 3-necked RB flask equipped with a stir bar and a thermometer is charged with 10.0g of (2*S*, 3*R*)-1-(Dimethylamino)-3-(3-methoxyphenyl)-2-methylpentan-3-ol, followed by 80 ml DCM and 12.7 ml triethylchlorosilane. The mixture is then cooled under stirring to 0-5 °C. 10.61 g of aluminium trichloride are added portionwise. Reaction mixture is stirred at 0-5 °C for 1-2 hours. Upon completion of reaction (TLC), 100 ml of DM water are added and mixture is stirred for 30 minutes at ambient temperature. Organic phase is separated and solvent is stripped off. Residue is partitioned between 50 ml t-butyl methyl ether and 10 ml HCl 1.0 N. Aqueous phase is transferred to 500 ml RB flask and 7.0 ml NaOH 50% w/v are added, followed by 50 ml DCM. Organic phase is separated, dried over sodium sulfate and filtered. Solvent is stripped off to provide 8.53 g of crude product. HPLC: 81.2% for the two diastereomers (IIa and IIb).

### EXAMPLE 6:

A 250 ml 3-necked RB flask equipped with a stir bar and a thermometer is charged with 10.0g of (2S, 3R)-1-(Dimethylamino)-3-(3-methoxyphenyl)-2-methylpentan-3-ol hydrochloride salt, followed by 80 ml DCM and 22.2 ml triethylchlorosilane. The mixture is then cooled under stirring to 0-5 °C. 11.58 g of aluminium trichloride are added portionwise. Reaction mixture is stirred at ambient temperature for 1-2 hours. Upon completion of reaction (TLC), reaction mixture is poured into an aq. solution of 100 ml sodium potassium tartrate 20% w/v and stirred for a few minutes. Then 25 ml of 50% w/v aq. solution of sodium hydroxide is added and the mixture is filtered. Organic phase is collected. Aqueous phase is extracted with 50 ml DCM. Organic phase is separated, combined with previous one and solvent is stripped off. To the residue 50 ml t-butyl methyl ether are added, followed by 80 ml of 1.0 N HCl. Aqueous phase is collected, 9.0 ml aq. solution 50% w/v sodium hydroxide is added and aq. phase is extracted twice with 50 ml dichloromethane. Combined organic phases are dried over sodium sulfate, filtered and solvents are stripped off to provide 7.9 g of crude product. HPLC: 98.1% for the two diastereomers (IIa and IIb).

### EXAMPLE 7:

A 25 ml RB flask equipped with a magnet bar is charged with 1.0 g of the crude product of example 6, followed by 5.0 ml butanone at ambient temperature. 0.59 mL of chlorotrimethylsilane are added followed by 84 µL of DM water. The mixture is further stirred for 1-2 hours and filtered through Buchner funnel. The wet cake is washed twice with 1.0 ml butanone and dried to afford 378 mg of (2R, 3R)-3-(3-methoxyphenyl)-*N,N*,2-trimethylpentane-1-amine hydrochloride salt. HPLC: 74.8% IIa, 23.7% compound of formula IIb.

### EXAMPLE 8:

A 25 ml RB flask equipped with a magnet bar is charged with 1.07 g of the crude product of example 6, followed by 8.0 ml butanone at ambient temperature. 40 µL of DM water are added, followed by dropwise addition of 280 µL of chlorotrimethylsilane. The mixture is further stirred for 2 hours and filtered through Buchner funnel. The wet cake is washed with 2.0 ml butanone and dried to afford 290 mg of (2*R*, 3*R*)-3-(3-methoxyphenyl)-*N*,*N*,2-trimethylpentane-1-amine hydrochloride salt. HPLC: 87.0% IIa, 10.6% IIb.

### EXAMPLE 9:

A 25 ml RB flask equipped with a magnet bar is charged with 550 mg of the crude product of example 6, followed by 2.2 ml acetone at ambient temperature. 21 µL of DM water are added, followed by dropwise addition of 148 µL of chlorotrimethylsilane. The mixture is further stirred for 24 hours. 2.2 ml of t-butyl methyl ether are added and suspension forms. The mixture is stirred for 2 hours and then filtered through Buchner funnel. The wet cake is washed with 2.0 ml t-butyl methyl ether and dried to afford 190 mg of (2*R*, 3*R*)-3-(3-methoxyphenyl)-*N*,*N*,2-trimethylpentane-1-amine hydrochloride salt. HPLC: 91.2% compound of formula IIa and 7.7% compound of formula IIb.

### EXAMPLE 10:

A 50 ml RB flask equipped with a thermometer and a stirring bar is charged with 1.0 g of the crude product of example 6, followed by 1.5 ml aqueous methanol 10%. To the resulting solution is added 1.8 g di-p-touolyl-tartaric acid-L. The mixture is heated up to reflux for 30 minutes. It is then gradually cooled down to 5-10 °C and further stirred for 1.5 hours. Mixture is filtered under vacuum and the cake is washed with 2.0 ml aq. methanol 10%, to provide 1.78 g of (2R, 3R)-3-(3-methoxyphenyl)-N,N,2-trimethylpentane-1-amine hydrochloride with diastereomeric purity 75.8% compound of formula IIa and 22.0% compound of formula IIb as a white solid.

## Claims

1. A process for the preparation of a compound of formula **IIa** or salt thereof from compound of formula **IIIa** or a salt thereof, comprising the steps of:
a) performing deoxygenation of compound of formula **IIIa** or a salt thereof in the presence of a hydrosilane reagent and an acid, selected from Lewis acids or protic acids, to provide compound of formula **IIa**, optionally in the form of a diastereomeric mixture; 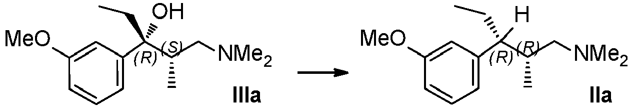
b) optionally treating the product of step a) with an acid, forming an acid addition salt via the amine group, selected from hydrochloric acid, hydrobromic acid, nitric acid, oxalic acid, succinic acid, maleic acid, fumaric acid, sulfuric acid, phosphoric acid, acetic acid, priopionic acid, benzenesulfonic acid, toluenesulfonic acid, citric acid, tartaric acid, di-aroyl-tartaric acid, malic acid, mandelic acid and its derivatives, camphorosulfonic acid and its derivatives and separating the salt of the desired (2R, 3R)-[3-(3-methoxyphenyl)-2-methylpentyl]dimethylamine (compound of formula **IIa**).

2. A process, according to claim 1, wherein the hydrosilane reagent is selected from triethylsilane, trimethylsilane, dimethylphenylsilane, phenyl silane, triphenylsilane, trichlorosilane, tris(trimethylsilyl)silane, polymethylhydrosiloxane.

3. A process, according to claims 1 or 2, wherein the acid of step a is selected from titanium tetrachloride, aluminium chloride, aluminium bromide, boron trifluoride, boron tribromide, tin tetrachloride, tin tetrabromide, stannous chloride, ferric chloride, zinc chloride, trifluoroacetic acid, p-toluolosulfonic acid or methanesulfonic acid.

4. A process, according to any preceding claim, wherein the acid in step b is selected from hydrochloric acid, hydrobromic acid, nitric acid, oxalic acid, succinic acid, maleic acid, fumaric acid, sulfuric acid, phosphoric acid, acetic acid, priopionic acid, benzenesulfonic acid, toluenesulfonic acid, citric acid, tartaric acid and its derivatives, malic acid, mandelic acid and its derivatives, camphorosulfonic acid and its derivatives.

5. A process, according to any preceding claim, wherein compound of formula IIIa is in the form of an acid addition salt.

6. A process, according to claim 5, wherein the acid addition salt is a hydrochloride or hydrobromide salt.

7. A process for the preparation of Tapentadol or a pharmaceutically acceptable salt thereof, comprising steps a and b as defined in any of the preceding claims and further comprising demethylating compound of formula **IIa**, to provide compound of formula **I** and optional conversion of compound of formula **I** into a pharmaceutically acceptable salt thereof.

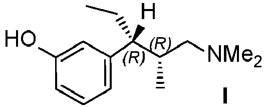

## Patentansprüche

1. Verfahren für die Zubereitung einer Verbindung der Formel **IIa** oder eines Salzes davon aus einer Verbindung der Formel **IIIa** oder aus einem Salz davon, welches die folgenden Schritte umfasst:
a) Desoxygenierung der Verbindung der Formel **IIIa** oder eines Salzes davon in Anwesenheit von einem Hydrosilan-Reagenz und einer Säure, ausgewählt aus Lewis-Säuren oder protischen Säuren, um die Verbindung der Formel **IIa**, gegebenenfalls in Form einer Diastereomer-Mischung, zu erhalten; 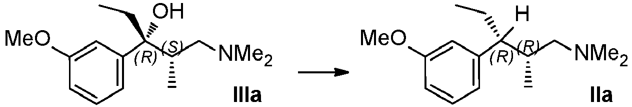
b) optionale Behandlung des Produkts aus Schritt a) mit einer Säure, damit ein Säureadditionssalz über die Aminogruppe, ausgewählt aus Salzsäure, Bromwasserstoffsäure, Salpetersäure, Oxalsäure, Bernsteinsäure, Maleinsäure, Fumarsäure, Schwefelsäure, Phosphorsäure, Essigsäure, Propionsäure, Benzolsulfonsäure, Toluolsulfonsäure, Zitronensäure, Weinsäure, Di-Aroylweinsäure, Äpfelsäure, Mandelsäure und ihre Derivate, Camphersulfonsäure und ihre Derivate, gebildet und das Salz des gewünschten (2R, 3R)-[3-(3-Methoxyphenyl)-2-methyl-pentyl]dimethylamins (Verbindung der Formel **IIa**) abgetrennt wird.

2. Verfahren nach Anspruch 1, **dadurch gekennzeichnet, dass** das Hydrosilan-Reagenz aus Triethylsilan, Trimethylsilan, Dimethylphenylsilan, Phenylsilan, Triphenylsilan, Trichlorsilan, Tris(trimethylsilyl)silan, Polymethylhydrosiloxan ausgewählt ist.

3. Verfahren nach Anspruch 1 oder 2, **dadurch gekennzeichnet, dass** die Säure aus Schritt a aus Titantetrachlorid, Aluminiumchlorid, Aluminiumbromid, Bortrifluorid, Bortribromid, Zinntetrachlorid, Zinntetrabromid, Zinn(II)-chlorid, Eisen(III)-chlorid, Zinkchlorid, Trifluoressigsäure, p-Toluolosulfonsäure oder Methansulfonsäure ausgewählt ist.

4. Verfahren nach einem der vorhergehenden Ansprüche, **dadurch gekennzeichnet, dass** die Säure in Schritt b aus Salzsäure, Bromwasserstoffsäure, Salpetersäure, Oxalsäure, Bernsteinsäure, Maleinsäure, Fumarsäure, Schwefelsäure, Phosphorsäure, Essigsäure, Priopionsäure, Benzolsulfonsäure, Toluolsulfonsäure, Zitronensäure, Weinsäure und ihren Derivaten, Apfelsäure, Mandelsäure und ihren Derivaten, Camphersulfonsäure und ihren Derivaten ausgewählt ist.

5. Verfahren nach einem der vorhergehenden Ansprüche, **dadurch gekennzeichnet, dass** die Verbindung der Formel IIIa in Form eines Säureadditionssalzes ist.

6. Verfahren nach Anspruch 5, **dadurch gekennzeichnet, dass** das Säureadditionssalz ein Hydrochlorid- oder Hydrobromidsalz ist.

7. Verfahren für die Zubereitung von Tapentadol oder eines pharmazeutisch verwendbaren Salzes davon, welches die Schritte a und b umfasst, wie diese in einem der vorhergehenden Ansprüche definiert wurden, und ferner die Demethylierung der Verbindung der Formel **IIa** umfasst, um die Verbindung der Formel I bereitzustellen, und optional Umwandlung der Verbindung der Formel **I** in ein pharmazeutisch verwendbares Salz davon.

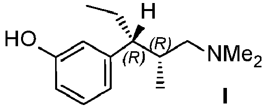

## Revendications

1. Procédé de préparation d'un composé de formule **IIa** ou d'un sel de celui-ci à partir d'un composé de formule **IIIa** ou d'un sel de celui-ci, comprenant les étapes consistant à :
a) effectuer la désoxygénation du composé de formule **IIIa** ou d'un sel de celui-ci en présence d'un réactif hydrosilane et d'un acide, choisi parmi les acides de Lewis ou les acides protiques, pour obtenir le composé de formule **IIa,** éventuellement sous la forme d'un mélange diastéréomérique ; 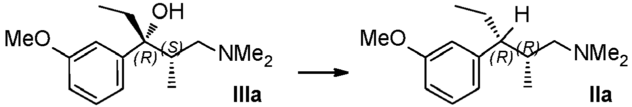
b) traiter, éventuellement, le produit de l'étape a) avec un acide, en formant un sel d'addition d'acide via le groupe amine, choisi parmi l'acide chlorhydrique, l'acide bromhydrique, l'acide nitrique, l'acide oxalique, l'acide succinique, l'acide maléique, l'acide fumarique, l'acide sulfurique, l'acide phosphorique, l'acide acétique, l'acide priopionique, l'acide benzènesulfonique, l'acide toluènesulfonique, l'acide citrique, l'acide tartrique, l'acide di-aroyl-tartrique, l'acide malique, l'acide mandélique et ses dérivés, l'acide camphorosulfonique et ses dérivés; et séparer le sel de la (2R,3R)-[3-(3-méthoxyphényl) -2-méthyl-pentyl]diméthylamine souhaitée (composé de formule **IIa**).

2. Procédé selon la 1ère revendication, dans lequel le réactif hydrosilane est choisi parmi le triéthylsilane, le triméthylsilane, le diméthylphénylsilane, le phénylsilane, le triphénylsilane, le trichlorosilane, le tris(triméthylsilyl)silane, le polyméthylhydrosiloxane.

3. Procédé selon la 1ère ou la 2ème revendication, dans lequel l'acide de l'étape (a) est choisi parmi le tétrachlorure de titane, le chlorure d'aluminium, le bromure d'aluminium, le trifluorure de bore, le tribromure de bore, le tétrachlorure d'étain, le tétrabromure de titane, le chlorure stannique, le chlorure ferrique, le chlorure de zinc, l'acide trifluoroacétique, l'acide p-toluolosulfonique ou l'acide méthanesulfonique.

4. Procédé selon l'une quelconque des revendications précédentes, dans lequel l'acide de l'étape (b) est choisi parmi l'acide chlorhydrique, l'acide bromhydrique, l'acide nitrique, l'acide oxalique, l'acide succinique, l'acide maléique, l'acide fumarique, l'acide sulfurique, l'acide phosphorique, l'acide acétique, l'acide priopionique, l'acide benzènesulfonique, l'acide toluènesulfonique, l'acide citrique, l'acide tartrique et ses dérivés, l'acide malique, l'acide mandélique et ses dérivés, l'acide camphorosulfonique et ses dérivés.

5. Procédé selon l'une quelconque des revendications précédentes, dans lequel le composé de formule IIIa revêt la forme d'un sel d'addition d'acide.

6. Procédé selon la 5ème revendication, dans lequel le sel d'addition d'acide est un sel de chlorhydrate ou de bromhydrate.

7. Procédé de préparation de Tapentadol ou d'un sel pharmaceutiquement acceptable de celui-ci, comprenant les étapes (a) et (b) telles que définies dans l'une quelconque des revendications précédentes et comprenant en outre la déméthylation du composé de formule **IIa**, pour fournir le composé de formule **I** et la conversion facultative du composé de formule **I** en un sel pharmaceutiquement acceptable de celui-ci.
